# EUROPEAN PATENT APPLICATION

(11) **EP 4 840 309 A2**
(43) Date of publication of application: **18.11.2026**
(21) Application number: 26201104.2
(22) Date of filing: 12.07.2024
(51) Int. Cl.: A01N 1/10

(54) **INCREASING VIABILITY OF CELLS WITHIN A FROZEN CELLULAR IMPLANT**

(30) Priority: 14.07.2023 US 202363513835 P
(62) Document number(s) of the earlier application(s) in accordance with Art. 76 EPC: 24843765.9 / 4 742 894
(71) Applicant: Advanced Biologics, LLC, Carlsbad, California 92008 (US)
(72) Inventor: GOVIL, Amit, Carlsbad, California, 92008 (US)
(74) Representative: Berggren Oy

(57) **Abstract**

The cryopreservation of cells from human tissue has challenges from both logistical timing as well as maintenance of cell viability. Therefore, needed are methods to increase cell viability in cellular tissue implants that allow for greater flexibility in timing for processing the implant. Therefore, disclosed herein is a method for increasing viability of cells within a frozen cellular implant, that involves soaking the cellular implant in a first cryoprotectant solution for at least 1 minute, soaking the cellular implant in a second cryoprotectant solution for at least 1 minute, and freezing the cellular implant. Also disclosed is a method for sterilizing a cellular implant comprised of collagen while maintaining cell viability that involves providing a cellular implant, adding a cryoprotectant to the cellular implant, freezing and maintaining a frozen temperature, and irradiating the cellular implant with radiation.

## Description

### CROSS-REFERENCE TO RELATED APPLICATIONS

This application claims benefit of U.S. Provisional Application No. 63/513,835, filed July 14, 2024, which is hereby incorporated herein by reference in its entirety.

### BACKGROUND OF THE INVENTION

The cryopreservation of cells from human tissue has challenges from both logistical timing as well as maintenance of cell viability. Therefore, needed are methods to increase cell viability in cellular tissue implants that allow for greater flexibility in timing for processing the implant.

### SUMMARY OF THE INVENTION

Disclosed herein is a method for increasing viability of cells within a frozen cellular implant, that involves soaking the cellular implant in a first cryoprotectant solution for at least 1 minutes, soaking the cellular implant in a second cryoprotectant solution for at least 1, 2, 3, 4, 5, 6, 7, 8, 9, or 10 minutes, and freezing the cellular implant to a temperature of from -10°C to -200°C, including from -10°C to -100°C, -70°C to -200°C, and -70°C to -100°C.

In some embodiments the method further involves soaking the cellular implant in a third cryoprotectant solution prior to freezing. In some embodiments the method further involves soaking the cellular implant in a fourth cryoprotectant solution prior to freezing. It can be understood that there is no limit to the number of additional soaking steps as they will continue to dilute out any saline left within the cellular implant.

Also disclosed is a method for sterilizing a cellular implant comprised of collagen while maintaining cell viability that involves providing a cellular implant, adding a cryoprotectant to the cellular implant, freezing and maintaining a frozen temperature of from -10°C to -200°C, and irradiating the cellular implant with radiation from 1 Gy to 50 kGy. In some embodiments the radiation comprises gamma or electron beam irradiation.

In some embodiments the cellular implant of the disclosed methods is derived from bone, dermis, adipose, fascia, ligament, tendon, muscle, ocular tissue, amnion, umbilical cord, placental tissue, blood, bone marrow, or any component thereof. In some embodiments the cells that remain viable comprise progenitor cells, osteprogenitors, mesenchymal stem cells, fibroblasts, bone cells, or vascular cells. In some embodiments the cellular implant comprises an extracellular matrix that has been growth factor enriched or cellularly enriched.

In some embodiments the freezing rate of the disclosed methods is from 0.1°C/min to 100°C/min, including from 0.1°C/min to 10°C/min, from 1°C/min to 100°C/min, from 10°C/min to 100°C/min, and from 1°C/min to 10°C/min.

In some embodiments the cryoprotectant of the disclosed methods comprises glycerol, polyethylene glycol, or a combination thereof. In some embodiments the cryoprotectant is free of dimethyl sulfoxide (DMSO) and contains saccharide. In some embodiments the cryoprotectant comprises a saccharide, such as sucrose, sorbitol, glucose, fructose, galactose, trehalose, mannose, maltose, or combinations thereof.

In some embodiments the cellular implant of the disclosed methods is derived from bone, dermis, adipose, fascia, ligament, tendon, muscle, ocular tissue, amnion, umbilical cord, placental tissue, blood, bone marrow, or any component thereof.

In some embodiments the cellular implant contains cancellous allograft bone and/or cortical allograft bone. In some embodiments the cancellous allograft bone and/or cortical allograft bone is demineralized or partially demineralized. In some embodiments the cancellous allograft bone and/or cortical allograft bone is demineralized or partially demineralized while other portions of the cancellous allograft bone and/or cortical allograft bone remain mineralized. In some embodiments the implant comprises growth factor enriched acellular cancellous bone combined with cellular cancellous bone from the same donor.

### DETAILED DESCRIPTION

Before the present disclosure is described in greater detail, it is to be understood that this disclosure is not limited to particular embodiments described, and as such may, of course, vary. It is also to be understood that the terminology used herein is for the purpose of describing particular embodiments only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the preferred methods and materials are now described.

All publications and patents cited in this specification are herein incorporated by reference as if each individual publication or patent were specifically and individually indicated to be incorporated by reference and are incorporated herein by reference to disclose and describe the methods and/or materials in connection with which the publications are cited. The citation of any publication is for its disclosure prior to the filing date and should not be construed as an admission that the present disclosure is not entitled to antedate such publication by virtue of prior disclosure. Further, the dates of publication provided could be different from the actual publication dates that may need to be independently confirmed.

As will be apparent to those of skill in the art upon reading this disclosure, each of the individual embodiments described and illustrated herein has discrete components and features which may be readily separated from or combined with the features of any of the other several embodiments without departing from the scope or spirit of the present disclosure. Any recited method can be carried out in the order of events recited or in any other order that is logically possible.

Embodiments of the present disclosure will employ, unless otherwise indicated, techniques of chemistry, biology, and the like, which are within the skill of the art.

The following examples are put forth so as to provide those of ordinary skill in the art with a complete disclosure and description of how to perform the methods and use the probes disclosed and claimed herein. Efforts have been made to ensure accuracy with respect to numbers (e.g., amounts, temperature, etc.), but some errors and deviations should be accounted for. Unless indicated otherwise, parts are parts by weight, temperature is in °C, and pressure is at or near atmospheric. Standard temperature and pressure are defined as 20 °C and 1 atmosphere.

Before the embodiments of the present disclosure are described in detail, it is to be understood that, unless otherwise indicated, the present disclosure is not limited to particular materials, reagents, reaction materials, manufacturing processes, or the like, as such can vary. It is also to be understood that the terminology used herein is for purposes of describing particular embodiments only, and is not intended to be limiting. It is also possible in the present disclosure that steps can be executed in different sequence where this is logically possible.

It must be noted that, as used in the specification and the appended claims, the singular forms "a," "an," and "the" include plural referents unless the context clearly dictates otherwise.

As used interchangeably herein, "subject," "individual," or "patient," refers to a vertebrate, preferably a mammal, more preferably a human. Mammals include, but are not limited to, murines, simians, humans, farm animals, sport animals, and pets. The term "pet" includes a dog, cat, guinea pig, mouse, rat, rabbit, ferret, and the like. The term farm animal includes a horse, sheep, goat, chicken, pig, cow, donkey, llama, alpaca, turkey, and the like.

As used herein, "biocompatible" or "biocompatibility" refers to the ability of a material to be used by a patient without eliciting an adverse or otherwise inappropriate host response in the patient to the material or a derivative thereof, such as a metabolite, as compared to the host response in a normal or control patient.

As used herein, "cell," "cell line," and "cell culture" include progeny. It is also understood that all progeny may not be precisely identical in DNA content, due to deliberate or inadvertent mutations. Variant progeny that have the same function or biological property, as screened for in the originally transformed cell, are included.

As used herein, "autologous" refers to being derived from the same subject that is the recipient.

As used herein, "allograft" refers to a graft that is derived from one member of a species and grafted in a genetically dissimilar member of the same species.

As used herein "xenograft" or "xenogeneic" refers to a substance or graft that is derived from one member of a species and grafted or used in a member of a different species.

As used herein, "autograft" refers to a graft that is derived from a subject and grafted into the same subject from which the graft was derived.

As used herein, "allogeneic" refers to involving, derived from, or being individuals of the same species that are sufficiently genetically different so as to interact with one another antigenically.

As used herein, "syngeneic" refers to subjects or donors that are genetically similar enough so as to be immunologically compatible to allow for transplantation, grafting, or implantation.

As used herein, "implant" or "graft," as used interchangeably herein, refers to cells, tissues, or other compounds, including metals and plastics, that are inserted into the body of a subject.

As used herein, "donor" refers to a subject from which cells or tissues are derived.

### Cryopreservation

The temperature(s) suitable for freezing or storing the biological material may vary. For instance, cells may be frozen or stored at a temperature ranging from about -4 °C to about -200 °C. In some embodiments, cells may be frozen or stored at or above the boiling temperature of liquid nitrogen, i.e., at or above about -196 °C.

The cryoprotectant solution may comprise a buffer system (e.g., a physiological buffer). The present preservation composition may comprise a balanced salt solution or any physiological solution. Non-limiting examples of the buffer systems include phosphoric acid buffers (for example, phosphate buffered saline (PBS)), BES, TES, acetamidoglycine, glycine amides, glycylglycine, TRICINE, TALP, tris-ethanolamine, veronal, and HEPES. In certain embodiments, the concentration of the buffer in the present composition ranges from about 1 mM to about 1000 mM, from about 1 mM to about 200 mM, from about 5 mM to about 200 mM, or from about 5 mM to about 50 mM. Non-limiting examples of culture media include, Dulbecco's Modified Eagle Media (DMEM) , Minimal Essential Medium (MEM) , Knockout-DMEM (KO-DMEM) , Glasgow Minimal Essential Medium (G-MEM) , Basal Medium Eagle (BME) , DMEM/Ham's F12, Advanced DMEM/Ham's F12, Iscove's Modified Dulbecco's Media and Minimal Essential Media (MEM) , Ham's F-10, Ham's F-12, Medium 199, RPMI 1640 Media, and combinations thereof and/or modifications thereof.

In certain embodiments, the present composition has a pH ranging from about 6.0 to about 8.5, from about 6.5 to about 8, from about 6.9 to about 7.5, or from about 7.2 to about 7.4, at room temperature or ambient temperature (for example, at 25°C) .

In certain embodiments, the cryoprotectant solution is packaged in unit forms. In one embodiment, the cryopreservation composition is packaged in a volume of 10 ml, 50 ml, 100 ml, 500 ml or 1 L. In certain embodiments, the preservation composition is packaged as a 1X, 1.5X, 2X, 3X, 5X, 10X, or 20X solution.

In certain embodiments, when or before being mixed/combined with the biological material, the temperature of the cryopreservation composition ranges from about 2 °C to about 45 °C, from about 10 °C to about 40 °C, from about 15 °C to about 40 °C, from about 20 °C to about 40 °C, from about 30 °C to about 40 °C, from about 33 °C to about 38 °C, or about 37 °C.

In certain embodiments, the freezing container having the cryopreservation composition with the biological material is placed at a temperature of between 0 °C and -20 °C, - 20°C and -40°C, -40°C and -70°C, -70 °C and -100 °C, or -80 °C for a period of time (e.g., overnight). Thereafter, the container may be transferred to liquid nitrogen (N₂) at about -196 °C.

In certain embodiments, the freezing container having the cryoprotectant solution with the cellular implant is put at a temperature of between -60 °C and -100 °C, or at -80 °C for a period of time (e.g., overnight). Thereafter, the container may be transferred to liquid nitrogen (N₂) at about -196 °C. In certain embodiments, the cellular implant in the cryoprotectant solution is exposed to a temperature less than or equal to -80 °C (e.g., dry ice) , less than or equal to - 100 °C, -196 °C (e.g., liquid nitrogen), or -205 °C (e.g., slush nitrogen which is a mixture of liquid and solid nitrogen).

In some embodiments, the cellular implant is suspended in the cryoprotectant solution, the suspension thus prepared is dispensed into freezing tubes (e.g., cryotubes, cryovials, etc.), and the resulting tubes are placed directly in an ultra-low temperature freezer (e.g., at -80 °C) to freeze the biological material. In one embodiment, the biological material in the cryopreservation composition is frozen directly in a freezer at -80 °C. In another embodiment, the biological material in the cryopreservation solution composition is refrigerated at about 2-8C before being frozen at -20°C, and transferred to storage at about -60°C to -80°C.

In some embodiments, parameters of the freezing step and/or thawing step are optimized such that temperature ramp-up and/or ramp-down rates do not disrupt the integrity of the biological material and does not adversely affect the viability or function of the biological material post-thaw.

In some embodiments, a cellular implant in the cryoprotectant solution is cooled in a temperature ramp-down phase having a selected rate of temperature reduction. In some embodiments, a rate of temperature reduction in a temperature ramp-down phase is about 10 °C per minute, about 1 °C per minute, about 2 °C per minute, about 5 °C per minute, about 7 °C per minute, about 12 °C per minute, about 15 °C per minute, about 17 °C per minute, about 20 °C per minute, or rates within the values above. In some embodiments, a temperature ramp-down phase may include cooling the biological material at a rate of approximately 10 °C per 10 seconds, 10 °C per 20 seconds, 10 °C per 30 seconds, 10 °C per 40 seconds, 10 °C per 50 seconds, 10 °C per 60 seconds, 10 °C per 70 seconds, 10 °C per 80 seconds, 10 °C per 90 seconds, 10 °C per 100 seconds, 10 °C per 110 seconds, 10 °C per 120 seconds, 10 °C per 130 seconds, 10 °C per 140 seconds, 10 °C per 150 seconds, 10 °C per 160 seconds, 10 °C per 170 seconds, 10 °C per 180 seconds, 1 °C per 190 seconds, or 10 °C per 200 seconds.

In certain embodiments, a temperature ramp-down phase may include a flash freezing (e.g., maximal temperature reduction) step.

In certain embodiments, the freezing step of the present method comprises non-linear cooling. The non-linear cooling cryopreservation protocol can be executed using a bulk freezing unit or a cryomicroscopy apparatus or other suitable apparatus, including one with a programmable thermocycler, that can be programmed to cool cells according to a predetermined cooling profile.

In some embodiments, the cellular implant is optionally subjected to an intermediate storing temperature for a desired period of time. The intermediate storing temperature may range from about 0 °C to about -100 °C, from about -50 °C to about -60 °C, from about -60 °C to about -70 °C, from about -70 °C to about -80 °C, from about -80 °C to about -90 °C, from about -90 °C to about -100 °C, and overlapping ranges thereof.

For example, in some embodiments, the cellular implant is stored at an intermediate storing temperature for a period of time before transfer to longer term storage. For example, the cells may be maintained at an intermediate storing temperature overnight, or any other suitable period of time, before being transferred to liquid nitrogen for long term storage. Other temperatures may be used in other embodiments (e.g., storage at -20 °C, -30 °C, -40 °C, - 50 °C, -60 °C, etc.) In several embodiments, a multi-step "step-down" procedure with multiple (2, 3, 4, 5 or more) intermediate storing temperatures is used.

The freezing of cellular implant in the cryoprotectant solution may be done using a programmed freezer. The freezing of the biological material in the cryopreservation composition may be done without using a programmed freezer. The freezing may be directional freezing, stationary freezing, and the like. Non-limiting examples of the freezing methods include using a directional freezing device, using a mechanical freezer, using a stepwise freezing apparatus, slush freezing, freezing in cryogenic fluid, freezing in controlled rate freezers, using a liquid bath freezer, using a cold air freezer, etc.

Any freezing apparatus capable of providing prolonged sub-zero temperatures to maintain a cryopreserved state may be used. Freezing and storage may be carried out in the same apparatus, or a first freezing apparatus may be used prior to transfer of frozen samples to a long-term storage apparatus. In one embodiment, liquid nitrogen storage vessels are used. In certain embodiments, passive freezing methods involving more sophisticated cooling devices, such as the programmable, rate controlled Planer freezers (Planer Products) are used.

The cellular implant in the cryoprotectant solution may be stored in a cryopreserved state for any length of time until they are needed. When the cells are at the storage temperature, they may be stored for a desired period, such as about 1-5 hours, about 5-12 hours, about 12-24 hours, about 24-48 hours, about 48 hours, about 1 week, about 2 weeks, about 3 weeks, about 1 month, about 2 months, about 3 months, about 6 months, about 1 year, about 2 years, about 3 years, about 4 years, about 5 years, or longer.

The cellular implant can remain in a preserved state (e.g., a cryopreserved state) for periods of days, weeks, months or years, until the biological material is required. When required, the cryopreserved biological material is retrieved and thawed. Therefore, in certain embodiments, the present method further comprises the step of thawing the frozen composition, more particularly under conditions that maintain cell viability.

The present compositions and methods may allow for the preservation cryopreservation of cells, wherein the cells maintain a good viability after recovery.

As used herein, the term "viability" refers to the percentage of viable biological material (such as cells, e.g., based on the presence of DNA and/or an intact cell membrane system, or viable viruses) . In certain embodiments, viable biological material refers to a biological material comprising some viable cells or fractions of cells that are metabolically active or would become metabolically active after their release from the preservation state.

In certain embodiments, the post-thaw viability of the biological material (e.g., cells or viruses) is at least about 50%, at least about 55%, at least about 60%, at least about 65%, at least about 70%, at least about 75%, at least about 80%, at least about 85%, at least about 90%, or at least about 95%.

In certain embodiments, the present compositions and methods ensure that the cells display a limited amount of, or minimal, necrosis and apoptosis after thawing. In certain embodiments, necrosis and/or apoptosis is observed in less than about 25%, less than about 20%, less than about 15%, less than about 10%, less than about 5%, or less than about 1% of the cells.

The viability can be measured by any methods known in the art. In certain embodiments, the viability is measured using a Trypan blue internalization test or by measuring propidium iodide uptake. In certain embodiments, the viability is measured by assaying the ability of cells to attach efficiently (e.g., the attachment assays) . In certain embodiments, proliferation assays can be used to determine if the attached cells can proliferate as expected after cryopreservation. Attachment and proliferation efficiency can be compared to control cells which have not undergone cryopreservation.

There are various tests known in the art to determine the viability and function of the cells. In certain embodiments, these tests are dependent on the cell type and the desired use of the cell.

For stem cells or progenitor cells, the methods described herein may further ensure that the cells maintain their pluripotency. This can be established by the determination of expression of lineage-specific markers. For instance, functional characterization of the mesenchymal stem cells may include induction of adipogenic, osteogenic and chondrogenic differentiation in vitro using commercially available differentiation kits and RT-PCR to detect lineage specific expression of mRNA, indicative for adipogenic, osteogenic and chondrogenic differentiation potential. Similarly, the quality of the undifferentiated stem cells can be tested by isolation of mRNA and testing on cell-specific markers. In particular embodiments, the ability to differentiate into a cell of the specified lineage is maintained, i.e., does not significantly differ from unprocessed cells. The pluripotency of the embryonic stem (ES) cells can be tested using art known methods, including, for example, Oct4-GFP expression, elevated alkaline phosphatase expression, and SSEA-1 surface glycoprotein expression. Several in vitro methods can be applied to assess stem cell recovery after experimental treatment. These assessments may include, but are not limited to, membrane integrity, metabolic and other functional assays and/or colony growth in culture, and fluorescent assays, such as SYTO/EB. In certain embodiments, differentiation tests, immunophenotype characterization, and/or an inspection of the morphology may be used to assay stem cells and/or progenitor cells.

For cryopreservation of zygotes, cleavage rates can be determined after cryopreservation and compared to control groups to determine if there has been any cellular damage during the cryopreservation process. The viability of oocytes can be determined by examination of the morphological characteristics of the cells following cryopreservation. Morphologically viable oocytes exhibit intact zona pellucida and plasma membrane and refractive cytoplasm, while non-viable oocytes appear degenerated when visualized under a light microscope. The ultimate criterion for oocyte viability and function is their capability to be fertilized by healthy sperm in vitro and in vivo, followed by cleavage, blastocyst, and/or hatching or development of the fetus.

In certain embodiments, the present preservation compositions and methods, as well as the biological material recovered from preservation using the present preservation compositions and methods can be used for research and/or clinical application (e.g., cell-based therapies, transplantation, regenerative medicine, diagnostics and genetic testing, cell/tissue banking for surveillance, toxicity testing and for in vitro fertilization) .

Any type of cells or tissues may be preserved using the present compositions and methods.

In certain embodiments, the cells are mammalian cells, including, but not limited to, human cells, murine cells, porcine cells, canine cells, equine cells and bovine cells.

Biological materials may comprise, without limitation, any of the following: fibroblasts, stem cells, progenitor cells, whole blood or fractions thereof, red blood cells, white blood cells, umbilical cord blood or fractions thereof, umbilical cord blood cells, bone marrow, oocytes, sperm, ova, embryos, cartilage, ovary, heart, skin, kidney, liver, lung. In addition, such biological material may comprise cellular organisms, which may be eukaryotes or prokaryotes, including bacteria, and yeast, etc. Additionally, biological material may also comprise whole multi-cellular organisms that are capable of surviving cryopreservation such as nematodes.

The present compositions and methods may be used to preserve any types of cells, including, but not limited to, pancreatic islet cells, chondrocytes, cells of neural origin, cells of hepatic origin, cells of opthalmolic origin, cells of orthopedic origin, cells from connective tissues, and cells of reproductive origin, and cells of cardiac and cardiovascular origin.

Stem cells include adult stem cells, embryonic stem cells, induced pluripotent stem cells (iPSCs) , peripheral blood stem cells, umbilical cord blood stem cells, mesenchymal stem cells, stem cells derived from tissues and organs or other sources, including fetal and/or embryonic sources, as well as mixtures of stem cells with other cells and from different sources. Adult stem cells include bone marrow stem cells, hematopoietic stem cells, skin stem cells, ocular stem cells, neural stem cells, cardiac stem cells, etc.

In certain embodiments, the stem cells of endodermal origin are pulmonary epithelial stem cells, gastrointestinal tract stem cells, pancreatic stem cells or hepatic oval cells and/or progenitor cells thereof. In particular embodiments, the cells of urogenital origin are either categorized as mammary and prostatic gland stem cells or ovarian and testicular stem cells and/or progenitor cells thereof. In particular embodiments, the cells of mesodermal origin are bone marrow cells, hematopoietic stem cells, stromal stem cells or cardiac stem cells and/or progenitor cells thereof. In particular embodiments, the cells of ectodermal origin are neural stem cells, skin stem cells or ocular stem cells and/or progenitor cells thereof.

Cell types that may be cryopreserved using the compositions and methods of the present disclosure include, for example, differentiated cells, such as fibroblasts, epithelial cells, cardiomyocytes, hepatocytes, neural cells, epidermal cells, keratinocytes, hematopoietic cells, melanocytes, chondrocytes, B-cells, T-cells, erythrocytes, macrophages, monocytes, or muscle cells; and undifferentiated cells, such as embryonic, mesenchymal, or adult stem cells. The cells can be haploid, diploid, or tetraploid. Other cells include cells from the bladder, brain, esophagus, fallopian tube, heart, intestines, gallbladder, kidney, liver, lung, ovaries, pancreas, prostate, spinal cord, spleen, stomach, testes, thymus, thyroid, trachea, ureter, urethra, or uterus.

In further particular embodiments, the cells are obtained from adult brain, bone marrow, blood vessels, skeletal muscle, skin, teeth, heart, gut, liver, or other adult tissues. In particular embodiments, the cells are selected from the group consisting of endodermal, urogenital, mesodermal or ectodermal origin.

Tissues include cornea, cartilage, bone, skin, heart valves, Islets of Langerhans, embryos from humans, animals, fish, shellfish and plants, and ovarian tissues from humans and animals. The present compositions and methods may also preserve engineered tissues and tissue constructs.

In certain embodiments, the present compositions and methods can be used to cryopreserve oocytes or sperm in assisted reproductive technology, or for patients undergoing chemotherapy or radiation therapy. The method can also be used for the cryopreservation of stem cells, which can then be used as the basis of stem cell-based therapies, cell transplantation, tissue engineering, and regenerative medicine. The method can also be used to cryopreserve oocytes or sperm from an animal that is rare or at risk of becoming extinct for future use in assisted reproductive technologies for the preservation of the species. The method can further be used for animal husbandry purposes (e.g., the breeding and raising of animals) , for example, for the cryopreservation of embryonic stem cells, gametocytes, oocytes, or sperm from animals such as cows, pigs, and sheep.

Cryopreserved cells are useful for the treatment of a variety of diseases. For example, in several embodiments, ocular cells are used to treat ocular diseases including, but not limited to age related macular degeneration (wet or dry), diabetic macular edema, idiopathic choroidal neovascularization, or high myopia macular degeneration. In some ocular embodiments, RPE cells are used. In several embodiments, cardiac stem cells are used to treat cardiovascular disorders such as myocardial infarction, ischemic cardiac tissue damage, congestive heart failure, aneurysm, atherosclerosis-induced events, cerebrovascular accident (stroke), and coronary artery disease. In several embodiments, liver stem cells are used to treat liver disease such as hepatitis, cirrhosis, cancer, and the like. Diseases in other tissues, such as the kidney, lung, pancreas, intestine, bone and/or cartilage, and neural tissues, among others, may be treated with the methods and devices disclosed herein. In some embodiments, harvested bone marrow stem cells may be used to repopulate hematopoietic cells that are reduced due to leukemias, cancers, or therapies that reduce blood cell counts.

The present disclosure is also useful in various methods of treatment. Cellular therapy, or cell therapy, can generally encompass transplantation of human or animal cells to replace or repair damaged tissue and/or cells. Cell therapy has been used to rebuild damaged cartilage in joints, repair spinal cord injuries, strengthen a weakened immune system, treat autoimmune diseases, and help patients with neurological disorders such as Alzheimer's disease, Parkinson's disease, and epilepsy. Further uses have included treatment of a wide range of chronic conditions such as arteriosclerosis, congenital defects, and sexual dysfunction.

Cell therapy typically involves the injection of either whole cells or cell extracts that are xenogenic, allogenic (from another human donor), or autologous (wherein the cells are extracted from and transplanted back into the same patient).

The present compositions and methods can be used in applications where it is useful to store cells for a period of time for use in later cell therapies. This can include storage of a patient's own cells for later transplantation, as well as storage of a generic cell line (for example, an embryonic stem cell line for use in research or therapies).

Viruses or viral particles can be any viruses. In certain embodiments, the viruses or viral particles comprises adenoviruses, adeno-associated viruses, retroviruses, herpes viruses and the like. In certain embodiments, the viruses or viral particles are those which may be used in gene therapy.

### Irradiation

In some embodiments, the radiation step in the disclosed methods includes E-beam irradiation. In certain embodiments, the radiation includes gamma radiation. In some embodiments, the radiation is provided at a dose sufficient to sterilize the cellular implant after freezing. In some embodiments, the radiation is at a dose that is low enough to prevent loss of biological functions or otherwise damage the tissue.

E-beam radiation is a form of ionizing energy that is generally characterized by its low penetration and high dosage rates. The beam, a concentrated, highly charged stream of electrons, is generated by the acceleration and conversion of electricity. The electrons are generated by equipment referred to as accelerators, which are capable of producing beams that are either pulsed or continuous. As the product/material being sterilized passes beneath or in front of the electron beam, energy from the electrons is absorbed. This absorption of energy alters various chemical and biological bonds within the product/material. The energy that is absorbed is referred to as the "absorbed dose." It is this absorption of energy-or "dose delivery"-that destroys the reproductive cells of microorganisms, e.g., by destroying their DNA chains.

A variety of E-beam irradiators can be used in the methods described herein and are commercially available, e.g., the RHODOTRON TT300 (from IBA, Louvain-la-Neuve, France) and the AEB emitter (from AEB, Willington, Mass.).

A cellular implant can be exposed to low-dose E-beam irradiation for a time and in an amount sufficient to achieve sterilization. The dosage of E-beam irradiation required to sterilize a cellular implant can vary based on, e.g., the size of the tissue, tissue origin of the cellular graft, and the type and amount of microbial contaminant in, or suspected of being present in, the tissue sample.

A cellular implant can be subjected to a one-sided exposure of the electron beam until a sterilizing dose of radiation is absorbed. An "absorbed dose" of radiation is expressed in terms of kilograys (kGy), wherein one kilogray is equal to one thousand joules of energy deposited per kilogram of material. For example, the decellularized tissue can be irradiated until an absorbed dose of about 6 kGy or more (e.g., 7 kGy, 8 kGy, 9 kGy, 10, kGy, 12 kGy, 15 kGy, 20 kGy, 22 kGy, 25 kGy, 27 kGy, or up to 30 kGy, or fractional values of the foregoing) is achieved. In some embodiments, a decellularized tissue can be exposed to irradiation until an absorbed dosage of about 6 kGy to about 30 kGy, about 10 kGy to about 15 kGy, or about 10 kGy to about 20 kGy is achieved.

E-beam or gamma irradiation of a cellular implant can be carried out, e.g., by placing the implant in a suitable container, e.g., a glass or plastic container, and exposing the cellular implant to the radiation. For example, the cellular implant may be placed on a conveyor, which then passes through the electron beam or is exposed to the gamma source. The time of exposure to the radiation can be proportional to the dimensions of the tissue.

Dosage can also be determined with the use of radiochromic dye films. Such films can be calibrated, usually in a gamma field, by reference to a national standard.

In some embodiments, the methods can also include the step of, following the sterilization step, testing for the presence or amount of one or more microorganisms (e.g., viruses, bacteria, or protozoa) in the cellular implant. Methods for determining whether a cellular implant contains a microorganism are known in the art and include, e.g., a variety of PCR techniques (e.g., quantitative amplification of specific microbial DNA or RNA markers); enzyme-linked immunosorbent assays (ELISA) using antibodies specific for microbial proteins; plaque-assays; hemagluttination assays; or colony formation tests. Effective sterilization can also be determined using conventional microbiological techniques, such as, for example, the inclusion of suitable biological indicators in a radiation batch or contacting the tissue with a culture medium, and incubating the medium to determine sterility of the implant.

### Cellular Implant

In certain embodiments, the cellular implant can be produced from any collagen-containing soft tissue and musculoskeletal tissue (e.g., bone, dermis, fascia, pericardium, dura, umbilical cords, placentae, cardiac valves, ligaments, tendons, vascular tissue (arteries and veins such as saphenous veins), neural connective tissue, urinary bladder tissue, ureter tissue, or intestinal tissue). Moreover, in certain embodiments, the tissues in which the cellular implant are placed include essentially any tissue that can be remodeled by invading or infiltrating cells. Relevant tissues include, without limitation, skeletal tissues such as bone, cartilage, ligaments, fascia, and tendon. Other tissues in which any of the above allografts can be placed include, without limitation, skin, gingiva, dura, myocardium, vascular tissue, neural tissue, striated muscle, smooth muscle, bladder wall, ureter tissue, intestine, and urethra tissue.

Furthermore, in some embodiments, cellular implant can have been made from one or more individuals of the same species as the recipient of the cellular implant. Alternatively, in certain embodiments, cellular implant can have been made from a porcine tissue, or other animal source, and can be implanted in a human patient. Species that can serve as recipients of cellular implant and donors of tissues or organs for the production of the cellular implant include, without limitation, humans, non-human primates (e.g., monkeys, baboons, or chimpanzees), porcine, bovine, horses, goats, sheep, dogs, cats, rabbits, guinea pigs, gerbils, hamsters, rats, or mice. In certain embodiments, the donors are animals (e.g., pigs) that have been genetically engineered to lack the terminal galactose-α-1-3-galactose moiety, or alternatively, tissue samples from such animals can be treated with an agent (such as alpha-galactosidase) to remove all, or substantially all, of the terminal galactose-α-α-1-3-galactose moieties from the tissue.

In some embodiments, the cellular graft is a bone graft. In some embodiments, a portion of cancellous, corticocancellos and/or cortical bone, or any combination thereof, can be harvested from a donor. In some embodiments, the harvested material can be harvested in such a way as to retain as much bone marrow in the harvested sample as possible.

In some embodiments, cancellous bone may be exposed to a weak lysing agent (such as less than 1M acetic acid) that only partially lyses the cell population present. In this embodiment, the partial lysis releases growth factors and binds them to the bone while other cells, such as mesenchymal stem cells and progenitor cells, may still remain viable and attached to the bone. After exposure to the lysing agent, the harvested sample may be exposed to buffers or other solutions to substantially neutralize the pH of the mixture of the growth factors and the lysing agent. In some embodiments, it may be desired that the pH be acidic (*e.g.,* pH below 7) or basic (*e.g.,* pH above 7) to retain solubility of particular growth factors or bioactive agents. For example, bone morphogenetic proteins (particularly BMP-2, BMP-4, BMP-6, BMP-7, BMP-9, BMP-14, and other bone morphogenetic proteins 1-30) are more soluble at acid pH values under 7 than neutral or basic pH.

In some embodiments, the cellular implant involves soft tissue. As used herein, "soft tissues" includes any tissue except for bone and bone marrow, and includes, but is not limited to, adipose tissue, muscle, cartilage, skin, tendons, ligaments, fascia, skin, fibrous tissue, synovial membranes, connective tissue, nerves, blood vessels, blood, lymph, and any organ.

A number of embodiments of the invention have been described. Nevertheless, it will be understood that various modifications may be made without departing from the spirit and scope of the invention. Accordingly, other embodiments are within the scope of the following claims.

Unless defined otherwise, all technical and scientific terms used herein have the same meanings as commonly understood by one of skill in the art to which the disclosed invention belongs. Publications cited herein and the materials for which they are cited are specifically incorporated by reference.

Those skilled in the art will recognize, or be able to ascertain using no more than routine experimentation, many equivalents to the specific embodiments of the invention described herein. Such equivalents are intended to be encompassed by the following claims.

Some non-limiting embodiments of the invention can be described using the following numbered items:

Item 1: A method for increasing viability of cells within a frozen cellular implant, comprising
a) soaking the cellular implant in a first cryoprotectant solution for at least 1 minutes,
b) soaking the cellular implant in a second cryoprotectant solution for at least 1 minutes, and
c) freezing the cellular implant to a temperature of from -10°C to -200°C.

The method of item 1, further comprising soaking the cellular implant in a third cryoprotectant solution prior to step c).

The method of item 1, wherein the cellular implant is derived from bone, dermis, adipose, fascia, ligament, tendon, muscle, ocular tissue, amnion, umbilical cord, placental tissue, blood, bone marrow, or any component thereof.

The method of item 1, wherein the freezing rate is from 0.1°C/min to 100°C/min.

The method of item 1, wherein the cells that remain viable comprise progenitor cells, osteprogenitors, mesenchymal stem cells, fibroblasts, bone cells, or vascular cells.

The method of item 1, wherein the cellular implant comprises an extracellular matrix that has been growth factor enriched or cellularly enriched.

The method of item 1, wherein the cryoprotectant comprises glycerol, polyethylene glycol, or a combination thereof.

The method of item 1, wherein the cryoprotectant is free of dimethyl sulfoxide (DMSO) and contains saccharide.

The method of item 1, wherein the cryoprotectant comprises a saccharide.

The method of item 1, wherein the saccharide comprises sucrose, sorbitol, glucose, fructose, galactose, trehalose, mannose, maltose, or combinations thereof.

Item 2: A method for sterilizing a cellular implant comprised of collagen while maintaining cell viability, comprising
a) providing a cellular implant,
b) adding a cryoprotectant to the cellular implant,
c) freezing and maintaining a frozen temperature of from -10°C to -200°C, and
d) irradiating the cellular implant with radiation from 1 Gy to 50 kGy.

The method of item 2, wherein the cellular implant is derived from bone, dermis, adipose, fascia, ligament, tendon, muscle, ocular tissue, amnion, umbilical cord, placental tissue, blood, bone marrow, or any component thereof.

The method of item 2, wherein the radiation comprises gamma or electron beam irradiation.

The method of item 2, wherein the cryoprotectant comprises glycerol, polyethylene glycol, or a combination thereof.

The method of item 2, wherein the cryoprotectant is free of dimethyl sulfoxide (DMSO) and contains saccharide.

The method of item 2, wherein the cryoprotectant comprises a saccharide.

The method of item 2, wherein the saccharide comprises sucrose, sorbitol, glucose, fructose, galactose, trehalose, mannose, maltose, or combinations thereof.

The method of item 2, wherein the cryoprotectant is used at a final concentration of 20%-100%.

The method of item 2, wherein the freezing rate is from 0.1°C/min to 100°C/min.

The method of item 2, wherein the cells that remain viable comprise progenitor cells, osteprogenitors, mesenchymal stem cells, fibroblasts, bone cells, or vascular cells.

The method of item 2, wherein the cellular implant comprises an extracellular matrix that has been growth factor enriched or cellularly enriched.

The method of item 2, wherein the cellular implant comprises cancellous allograft bone and/or cortical allograft bone.

The method of item 2, wherein the cancellous allograft bone and/or cortical allograft bone is demineralized or partially demineralized.

The method of item 2, wherein cancellous allograft bone and/or cortical allograft bone is demineralized or partially demineralized while other portions of the cancellous allograft bone and/or cortical allograft bone remain mineralized.

The method of item 2, wherein cellular the implant comprises growth factor enriched acellular cancellous bone combined with cellular cancellous bone from the same donor.

## Claims

1. A method for sterilizing a cellular implant comprised of collagen while maintaining cell viability, comprising
a) providing a cellular implant,
b) adding a cryoprotectant to the cellular implant,
c) freezing and maintaining a frozen temperature of from -10°C to -200°C, and
d) irradiating the cellular implant with radiation from 1 Gy to 50 kGy.

2. The method of claim 1, wherein the cellular implant is derived from bone, dermis, adipose, fascia, ligament, tendon, muscle, ocular tissue, amnion, umbilical cord, placental tissue, blood, bone marrow, or any component thereof.

3. The method of claim 1, wherein the radiation comprises gamma or electron beam irradiation.

4. The method of claim 1, wherein the cryoprotectant comprises glycerol, polyethylene glycol, or a combination thereof.

5. The method of claim 1, wherein the cryoprotectant is free of dimethyl sulfoxide (DMSO) and contains saccharide.

6. The method of claim 1, wherein the cryoprotectant comprises a saccharide.

7. The method of claim 6, wherein the saccharide comprises sucrose, sorbitol, glucose, fructose, galactose, trehalose, mannose, maltose, or combinations thereof.

8. The method of claim 1, wherein the cryoprotectant is used at a final concentration of 20%-100%.

9. The method of claim 1, wherein the freezing rate is from 0.1°C/min to 100°C/min.

10. The method of claim 1, wherein the cells that remain viable comprise progenitor cells, osteprogenitors, mesenchymal stem cells, fibroblasts, bone cells, or vascular cells.

11. The method of claim 1, wherein the cellular implant comprises an extracellular matrix that has been growth factor enriched or cellularly enriched.

12. The method of claim 1, wherein the cellular implant comprises cancellous allograft bone and/or cortical allograft bone.

13. The method of claim 12, wherein the cancellous allograft bone and/or cortical allograft bone is demineralized or partially demineralized.

14. The method of claim 13, wherein cancellous allograft bone and/or cortical allograft bone is demineralized or partially demineralized while other portions of the cancellous allograft bone and/or cortical allograft bone remain mineralized.

15. The method of claim 12, wherein the cellular implant comprises growth factor enriched acellular cancellous bone combined with cellular cancellous bone from the same donor.
